# EUROPÄISCHE PATENTSCHRIFT

(11) **EP 2 406 203 B1**
(45) Veröffentlichungstag und Bekanntmachung des Hinweises auf die Patenterteilung: **19.10.2016**
(21) Anmeldenummer: 10708915.3
(22) Anmeldetag: 26.02.2010
(51) Int. Cl.: C07C 5/327, C07C 5/333, B01D 53/78, B01D 53/77, B01D 53/14

(54) **VERFAHREN UND VORRICHTUNG ZUR VERMINDERUNG VON OLEFINVERLUSTEN BEI DER ENTFERNUNG VON KOHLENDIOXID AUS EINEM OLEFINSTROM AUS DEHYDRIERUNGSREAKTIONEN**
PROCESS AND APPARATUS FOR REDUCING OLEFIN LOSS DURING SEPARATION OF CARBON DIOXIDE FROM AN OLEFINSTREAM OBTAINED BY DEHYDRATION REACTIONS
PROCEDE ET DISPOSITIF POUR REDUIRE LA PERTE D'OLEFINE LORS DE LA SEPARATION DU DIOXYDE DE CARBONE D'UN COURANT D'OLEFINE OBTENU PAR DES PROCEDES DE DESHYDRATATION

(30) Priorität: 12.03.2009 DE 102009012452
(43) Veröffentlichungstag der Anmeldung: 18.01.2012
(73) Patentinhaber: ThyssenKrupp Industrial Solutions AG, 45143 Essen (DE)
(72) Erfinder: MENZEL, Johannes, 45731 Waltrop (DE)
(74) Vertreter: Kilchert, Jochen
(86) Internationale Anmeldenummer: PCT/EP2010/001196
(87) Internationale Veröffentlichungsnummer: WO 2010/102729

(56) Entgegenhaltungen:
- WO-A2-2006/094938
- US-B1- 6 290 754

## Beschreibung

Die Erfindung richtet sich auf ein Verfahren zur Verminderung von Alkenverlusten bei der Dehydrierung von Alkanen zu Alkenen, wobei Kohlendioxid als Nebenprodukt bei der Dehydrierung anfällt und wobei das entstandene Kohlendioxid mittels eines Absorptionsprozesses aus dem durch die Dehydrierungsreaktion entstehenden Rohgas entfernt wird.

Die Herstellung von Olefinen und insbesondere die Herstellung von gasförmigen, niederen Olefinen erfolgt in der Technik durch Dehydrierung von niederen Alkanen. Bei der Herstellung von Olefinen wird das Alkan mit einem Inertgas, typischerweise mit Wasserdampf, vermischt und durch einen Dehydrierungsreaktor geleitet. Dieser ist mit einem Katalysator beschickt, der die Alkandehydrierung katalysiert. Durch die Reaktion entsteht das gewünschte Alken, das nach der Reaktion üblicherweise in dem Produktgas in einem Anteil von wenigen Volumenprozent vorhanden ist. Bei der Reaktion entstehen Nebenprodukte, so dass das Produktgas neben dem erwünschten Olefin, dem Reaktionsprodukt Wasserstoff und dem vorher beigemischten Inertgas noch geringere Anteile an Kohlenmonoxid und an Kohlendioxid enthält.

Einen typischen Prozess zur Dehydrierung von Propan zu Propylen beschreibt die WO 2006050957 A1. Hierbei wird ein erstes Gasgemisch, welches Propan, Wasserstoff und Wasserdampf als inertes Gas enthält, in mindestens ein Katalysatorbett geleitet, welches übliche Dehydrierungsbedingungen aufweist. Da Dehydrierungsreaktionen häufig mit einer selektiven Wasserstoffverbrennung kombiniert werden, mit der der gebildete Wasserstoff zur Entfernung aus dem Gleichgewicht verbrannt wird, wird ein weiteres, Propan und Sauerstoff enthaltendes Gasgemisch, bei dem der Propangehalt gegenüber dem Sauerstoffgehalt überwiegt, in die gleiche Reaktionsvorrichtung geleitet, wo es mit dem ersten Gasgemisch unter Bildung von Propylen, Wasserdampf und Wasserstoff reagiert. Typische Temperaturen zur Ausführung der Reaktion sind 540 °C bis 820 °C bei Drücken von 40 bis 50 bar.

Die WO 2006/094938 A2 beschreibt ein Verfahren zur Herstellung von Propen aus Propan, in welchem ein Propan enthaltender Einsatzgastrom bereitgestellt wird, und der Propan enthaltende Einsatzgastrom, gegebenenfalls Wasserdampf und gegebenenfalls ein sauerstoffhaltiger Gasstrom, in eine Dehydrierzone eingespeist werden, und Propan einer Dehydrierung zu Propen unterworfen wird, wobei ein Produktgasstrom enthaltend Propan, Propen, Methan, Ethan, Ethen, Stickstoff, Kohlenmonoxid, Kohlendioxid, gegebenenfalls Wasserstoff und gegebenenfalls Sauerstoff erhalten wird, und der Produktgasstrom abgekühlt wird, und gegebenenfalls verdichtet wird, und Wasserdampf durch Kondensieren abgetrennt wird, und ein an Wasserdampf abgereicherter Produktgasstrom erhalten wird, und nicht kondensierbare oder leicht siedende Gasbestandteile durch Inkontaktbringen des Produktgasstroms mit einem inerten Absorptionsmittel und anschließender Desorption der in dem inerten Absorptionsmittel gelösten Gase abgetrennt werden, und wobei ein C3-Kohlenwasserstoffstrom und ein Abgasstrom enthaltend Propan, Propen, Methan, Ethan, Ethen, Stickstoff, Kohlenmonoxid, Kohlendioxid, gegebenenfalls Wasserstoff und gegebenenfalls Sauerstoff erhalten werden, in dem der C3-Kohlenwasserstoffstrom weiter aufgearbeitet wird. Die Desorption wird durch einen "Strippgasstrom" ausgeführt, welcher beispielhaft Stickstoff, Luft, Wasserdampf oder Propan/Propen-Gemische enthält. Die Verwendung von preisgünstigem Brenngas als "Strippgas", welches typischerweise Erdgas ist, wird nicht beschrieben.

Bevorzugt handelt es sich bei dem inerten Gas um Wasserdampf. Es ist jedoch auch möglich, beispielhaft Kohlendioxid oder Wasserstoff zu verwenden, oder jedes beliebige weitere Gas zu verwenden, welches sich als inertes Verdünnungsgas zur Durchführung der Reaktion eignet. Nach dem Herstellungsprozess durch Dehydrierung wird das erhaltene Produktolefin gekühlt und getrocknet. Der Trocknungsprozess kann beliebig geartet sein. Bevorzugt handelt es sich bei dem Trocknungsprozess um einen Kondensationsprozess. Der bei der Dehydrierung anfallende Wasserstoff muss in weiteren Prozessschritten von dem gewünschten Endprodukt, nämlich den Alkenen, abgetrennt werden. Die Abtrennung erfolgt vorzugsweise bei tieferen Temperaturen und erhöhten Drücken.

Nach der Wasserstoffentfernung wird das Rohgas komprimiert. Um den Verlust an Alkenen, die mit dem abgetrennten Wasserstoff die nachgeschaltete Trenneinrichtung über Kopf verlassen, möglichst gering zu halten, ist eine sogenannte "Cold-Box" vorgesehen, mittels der die im Wasserstoff enthaltenen Kohlenwasserstoffe, insbesondere die Alkene, zum größten Teil auskondensiert werden. Das Prozessgas erfährt dabei eine Temperaturabsenkung auf unter -100 °C. Um zu vermeiden, dass das Kohlendioxid bei diesen Temperaturen im Prozessgasweg desublimiert, was zu einer Zusetzung der Cold-Box führen würde, ist es notwendig, Kohlendioxid aus dem Rohgas soweit zu entfernen, dass es an keiner Stelle zu einer Feststoffbildung von Kohlendioxid kommen kann. Ebenso ist es notwendig, noch vorhandene Restgehalte von Schwefelverbindungen, die mit dem Einsatzgas in den Prozess gelangen, aus dem Rohgas zu entfernen.

Zu diesem Zwecke wird das gekühlte und getrocknete Gas einer Vorrichtung, die zur Entfernung von Sauergaskomponenten geeignet ist, zugeführt. Dies ist bevorzugt eine Kolonne, die eine Kontaktaufnahme eines sauergasabsorbierenden Lösungsmittels mit dem zu reinigenden Gas unter Druck ermöglicht. Das angereicherte Lösungsmittel, das die Gaswaschkolonne am Sumpf verlässt, wird zur Rückgewinnung von mitabsorbierten Kohlenwasserstoffen in einem sogenannten Hochdruck-"Flash"-Behälter entspannt. Der größte Mengenanteil an saurem Gas entfällt auf Kohlendioxid, welches zum einen Teil bereits in dem Ausgangsgas enthalten ist und welches sich zum anderen Teil bei der Reaktion mit dem in dem Ausgangsgas enthaltenen Sauerstoff aus den Kohlenwasserstoffen bildet.

Das in dem Hochdruck-Flash-Behälter teilentspannte Lösungsmittel wird einer weiteren Trennkolonne zugeführt, in der das im Lösungsmittel enthaltene Sauergas vom Lösungsmittel abgetrennt wird. Diese weitere Trennkolonne wird üblicherweise als Lösungsmittelregenerationskolonne bezeichnet. Das Lösungsmittel wird gegebenenfalls mittels Filtereinrichtungen gereinigt, und mittels einer Kreislaufpumpe wieder auf den Kopf der Sauergasabsorptionskolonne zurückgeführt.

Der die Sauergasabsorptionskolonne verlassende Kohlenwasserstoffstrom, bestehend aus leichten, gasförmigen Olefinen und Wasserstoff, wird mittels Tieftemperaturdestillationseinheiten und Ausfriervorrichtungen weiter aufgetrennt. Beispiele für Tieftemperaturdestillationsvorrichtungen sind C3-Splitter und Demethanisierer. Beispiele für Ausfriereinrichtungen sind sogenannte "Cold-Boxes".

Verfahren, die eine Abtrennung von restlichem Kohlendioxid aus dem Hochdruck-Flash-Behälter ermöglichen, sind bekannt. Dies sind beispielhaft Verfahren nach dem Stand der Technik, die eine Gaswäsche für den Kohlenwasserstoffstrom durchführen. Diese sind jedoch aufwendig und kostenintensiv, da diese letztlich eine zusätzliche Gaswäsche darstellen. Die in den Hochdruck-Flash-Behälter vorgenommene Entspannung wird aus diesem Grund so ausgeführt, dass der größte Teil des absorbierten Kohlendioxids im Lösungsmittel verbleibt.

Da bei der Entspannung in dem Hochdruck-Flash-Behälter keine vollständige Trennung der Kohlenwasserstoffe aus dem Lösungsmittel möglich ist, ohne dass auch das Kohlendioxid zum größten Teil dort freigesetzt wird, gelangt ein gewisser Anteil an mitabsorbierten Kohlenwasserstoffen in die Regenerationskolonne, in der die Sauergase nahezu vollständig vom Lösungsmittel abgetrennt werden. Die auch noch im Lösungsmittel enthaltenen Kohlenwasserstoffe verlassen zusammen mit den abgetrennten Sauergasen, hauptsächlich bestehend aus Kohlendioxid, die Regenerationskolonne als Abgas über Kopf.

Dies führt zu Verlusten an Kohlenwasserstoffen und insbesondere an Olefinen, da diese nur unter sehr hohem Aufwand von den Sauergaskomponenten getrennt werden können. In Lösungsmitteln, die zur Entfernung von Sauergasen verwendet werden, lösen sich insbesondere Alkene sehr gut. Ist der zu gewinnende Kohlenwasserstoff beispielsweise Propylen, so geht durch den Ausschleusungsprozess ein nennenswerter Teil des mitabsorbierten Propylens bei der Regeneration des Lösungsmittels zusammen mit dem entfernten Kohlendioxid verloren. Eine weitere Rückgewinnung von Propylen aus diesem Abgas ist mit hohen Kosten verbunden. Dies ist für den gesamten Prozess wirtschaftlich ungünstig.

Es ist daher eine Aufgabe der Erfindung, einen Prozess zur Verfügung zu stellen, der eine verbesserte Trennung von Kohlendioxid und Kohlenwasserstoffen bei der Sauergasentfernung ermöglicht.

Die Erfindung löst diese Aufgabe durch einen Prozess, der in den Sumpf eines Stoffaustauschelemente, wie Füllkörper und Packungen, enthaltenden Hochdruck-Flash-Behälters ein Brenngas aufgibt, wobei dieses Gas im Gegenstrom zu dem auf den Kopf des Hochdruck-Flash-Behälters aufgegebenen chemisch wirkenden absorbierenden Lösungsmittel in Form einer wässrigen Aminlösung, welches auch mitabsorbierte Kohlenwasserstoffe enthält, geführt wird und wobei der größte Teil der im Lösungsmittel noch enthaltenen Kohlenwasserstoffe aus dem chemisch wirkenden absorbierenden Lösungsmittel entfernt wird, und wobei durch die entsprechende Wahl von Temperatur und Druck das ebenfalls im Lösungsmittel enthaltene Kohlendioxid nur in geringerem Maße desorbiert wird, und es sich bei dem Brenngas um Erdgas handelt, welches für die Beheizung des Dehydrierungsreaktors verwendet wird und welches abgezweigt wird. Das aus dem Hochdruck-Flash-Behälter gewonnene Desorptionsgas wird in einer Ausführungsform der Erfindung wieder in den Rohgasstrom vor den Kompressor zurückgeführt, sodass die zurückgewonnenen Kohlenwasserstoffe vollständig für die weitere Produktaufbereitung zur Verfügung stehen.

Als Brenngas wird Erdgas verwendet. Dieses wird zum Beheizen des Dehydrierungsreaktors genutzt und wird als abgezweigter Teilstrom zum Durchströmen des Hochdruck-Flash-Behälters verwendet. Das als Hauptkomponente im Erdgas enthaltene Methan wird zusammen mit dem Wasserstoff aus dem Alkengewinnungsprozess ausgeschleust und findet als Heizgas weitere Verwendung im Gesamtprozess.

Die erfindungsgemäße Durchströmung des Hochdruck-Flash-Behälters, der in einer bevorzugten Ausführungsform der Erfindung als Füllkörperkolonne geartet ist, mit Erdgas als Strippgas erbringt eine deutliche Verbesserung der Desorption des aus der Dehydrierungsreaktion erhaltenen Kohlenwasserstoffes aus dem chemisch wirkenden absorbierenden Lösungsmittel zur Gaswäsche. In einer bevorzugten Ausführungsart der Erfindung wird der erhaltene Kohlenwasserstoff rekomprimiert und in den Prozess zurückgeführt. Durch die Ausführung des erfindungsgemäßen Verfahrens kann die Ausbeute an erhaltenem Olefin einer Dehydrierungsreaktion wesentlich gesteigert werden.

Beansprucht wird insbesondere ein Verfahren zur Entfernung von Kohlendioxid aus einem Kohlenwasserstoffstrom bei der Herstellung von Alkenen aus Alkanen, wobei
- ein Alkane und ein inertes Gas enthaltendes technisch sauerstofffreies Gasgemisch in eine mindestens ein Katalysatorbett enthaltende Reaktionsvorrichtung zur Dehydrierung des eingesetzten Alkans geleitet wird, und
- das gebildete Alkene, Alkane, Wasserstoff, das inerte Gas und Kohlendioxid (CO2) enthaltende Gasgemisch aus der Reaktionsvorrichtung abgezogen wird, und
- die im Prozessgas noch enthaltenen Sauergasbestandteile, wie Kohlendioxid, Schwefelwasserstoff (H2S), Carbonylsulfid (COS) und Merkaptane in einer der Dehydrierung nachgeschalteten Gaswäsche vollständig oder nahezu vollständig mittels eines chemischen Lösungsmittels absorbiert werden,
   **und das dadurch gekennzeichnet ist, dass**
- das gebildete Adsorbat, welches auch mitabsorbierte Kohlenwasserstoffe enthält, auf den Kopf eines Hochdruck-Flash-Behälters geleitet wird, welcher Stoffaustauschelemente enthält, und dem Sumpf des Hochdruck-Flash-Behälters ein Brenngas zugeführt wird, welches im Gegenstrom zu der über die Stoffaustauschelemente herabfließenden, angereicherten Absorptionslösung geführt wird, wobei der größte Teil der in der angereicherten Absorptionslösung enthaltenen Kohlenwasserstoffe und im besonderen die noch gelösten Alkene aus der Lösung desorbiert werden und den Hochdruck-Flash-Behälter über Kopf verlassen,
- es sich bei dem Brenngas um das Heizgas handelt, welches für die Beheizung des Dehydrierungsreaktors verwendet wird und welches abgezweigt wird, und es sich bei dem Heizgas, welches für die Beheizung des Dehydrierungsreaktors verwendet wird, um Erdgas handelt, und als Lösungsmittel für die Gaswäsche ein chemisch wirkendes Absorptionsmittel eingesetzt wird, und
- dass als chemisches Lösungsmittel für die Gaswäsche eine wässrige Aminlösung eingesetzt wird.

Da das den Hochdruck-Flash-Behälter über Kopf verlassende Strippgas ein kohlenwasserstoffhaltiges Gas mit einer ähnlichen Zusammensetzung wie das die Dehydrierungseinrichtung verlassende Prozessgas ist, kann dieses in einer vorteilhaften Ausführungsform der Erfindung zur Erhöhung der Effizienz als Heizgas weitere Verwendung im Gesamtprozess finden.

Bei dem für die Dehydrierung verwendeten Alkan handelt es sich in einer bevorzugten Ausführungsform um Propan. Dies wird in der Dehydrierungseinrichtung entsprechend dem Reaktionsgleichgewicht teilweise zu Propylen umgesetzt. Es kann sich bei dem verwendeten Einsatzgas auch um n-Butan, i-Butan oder um n-Pentan handeln. Dabei erhält man n-Buten, i-Buten oder n-Penten als Produktkohlenwasserstoffe. Prinzipiell kann jeder dehydrierbare Kohlenwasserstoff für den Prozess verwendet werden.

Der Druck des Hochdruck-Flash-Behälters ist dabei in bevorzugter Weise höher als der Druck an dem Einbindepunkt, zu dem das den "Hochdruck-Flash-Behälter" über Kopf verlassende Flashgas zurückgeführt wird. Ein bevorzugter Einbindepunkt ist dabei der Prozessgasweg hinter der Dehydrierungseinrichtung. Das Brenngas für die Strippung ist in bevorzugter Weise Erdgas, welches auch als Heizgas für die Dehydrierung verwendet wird.

Der Hochdruck-Flash-Behälter wird bei einem Druck von 1 bis 20 bar betrieben. Bevorzugt wird der Hochdruck-Flash-Behälter bei einem Druck von 2 bis 12 bar und besonders bevorzugt bei einem Druck von 4 bis 8 bar betrieben. Die Temperatur des auf den Kopf des Hochdruck-Flash-Behälters aufgegeben Lösungsmittels, wird dabei zwischen 40 °C bis 160 °C und bevorzugt auf eine Temperatur von 80 °C bis 120 °C eingestellt.

Als Lösungsmittel für die Gaswäsche eignen sich Lösungsmittel, die nach dem Stand der Technik häufig verwendet werden und die eine gute Absorptionsfähigkeit für Kohlendioxid besitzen. Erfindungsgemäß werden chemisch wirkende Absorptionsmittel in Form von wässrigen Aminlösungen, insbesondere von Aminverbindungen wie beispielweise Monoethanolamin oder Methyldiethanolamin, verwendet. Diese können auch einen sogenannten Aktivator enthalten. Die US 6290754 B1 beschreibt ein Verfahren zur Entsäuerung von Gasen, die flüssige Kohlenwasserstoffe enthalten, wobei das Gas in einer Absorptionszone mit einem flüssigen Absorptionsmittel behandelt wird, welches Methyldiethanolamin (MDEA) und einen Aktivator enthält, so dass man eine CO2-reiche Gasfraktion und einen regenerierten Lösungsmittelstrom erhält, und das regenerierte Lösungsmittel in die Absorptionszone zurückgeführt wird, wobei der Aktivator ein Aminoalkylaminoethanol ist und der Alkylrest 1 bis 4 Kohlenstoffatome enthält. Dieses Verfahren und die darin verwendete Lösungsmittelkombination können beispielhaft für den vorliegenden Prozess genutzt werden.

Das Lösungsmittel wird am Sumpf des Hochdruck-Flash-Behälters abgezogen und in eine Lösungsmittelregenerationskolonne gegeben. Das Lösungsmittel soll in erster Linie die Sauergaskomponenten, wie Schwefelwasserstoff (H2S), Merkaptane, Carbonylsulfid (COS) und insbesondere Kohlendioxid (CO2), selektiv gegenüber den Kohlenwasserstoffen aus dem Prozessgas entfernen. Diese verlassen nach der Desorption die Lösungsmittelregenerationskolonne über Kopf und können nach bedarfsweiser Kühlung weiterverwendet oder entsorgt werden. Es können alle Lösungsmittelregenerationskolonnen aus dem Stand der Technik verwendet werden, die für eine übliche Lösungsmittelregeneration geeignet sind. Diese können beispielhaft durch einen Reboiler beheizt werden. Das Lösungsmittel wird nach der Regeneration im Kreis zurück in die Absorptionskolonne geführt, wobei es zum Erwärmen des beladenen Lösungsmittels vor Eintritt in den Hochdruck-Flash-Behälter über einen Wärmetauscher geleitet werden kann. Bedarfsweise wird das regenerierte Lösungsmittel vor der Wiederverwendung gekühlt.

Das gereinigte Produktgas aus der Sauergaswäsche wird einer "Cold-Box" und einer Rückgewinnungskolonne zur weiteren Rückgewinnung von noch im Prozessgas enthalten Alkenen zugeführt. Die Abtrennung erfolgt dabei über Tieftemperaturtrennprozesse.

Beansprucht wird auch eine Vorrichtung zur Ausführung des erfindungsgemäßen Verfahrens. Hierzu gehört zunächst eine Absorptionskolonne, die eine Gaswäsche durch Inkontaktnahme mit einem sauergasabsorbierenden Lösungsmittel ermöglicht. Zur Ausführung des erfindungsgemäßen Verfahrens kommt eine Vorrichtung zur Anwendung, die eine Führung von Erdgas im Gegenstrom zu der auf den Kopf der Einrichtung aufgegebenen Lösung ermöglicht. Bevorzugt ist dies ein sogenannter Hochdruck-Flash-Behälter, der eine Entspannung des sauergasbeladenen Lösungsmittels ermöglicht und der mit Stoffaustauschelementen, vergleichbar einer Füllkörperkolonne, versehen ist. Die im Hochdruck-Flash-Behälter enthaltenen Elemente können stoffaustauschermöglichende Böden, Füllkörper, "Pall-Ringe", Raschig-Ringe, strukturierte Packungen oder Sattelkörper sein. Die Füllkörperkolonne enthält einen Aufgabestutzen für das sauergasbeladene Lösungsmittel, einen Aufgabestutzen für das Erdgas, einen Entnahmestutzen für ein sauergasbeladenes Lösungsmittel und einen Entnahmestutzen für einen gasförmigen Kohlenwasserstoffstrom. Zu der erfindungsgemäßen Vorrichtung gehören außerdem noch Kompressoren, Pumpen, Ventile, Rohrleitungen, Steuerungselemente, Filtereinrichtungen und Messeinrichtungen, je nach Anforderung.

Durch die Erfindung lassen sich Sauergase aus einem Kohlenwasserstoffstrom entfernen, wobei die Sauergase ohne einen wesentlich erhöhten Aufwand fast vollständig von Kohlenwasserstoffen befreit werden können. Die desorbierten Kohlenwasserstoffe können in einer vorteilhaften Ausführungsform in den Prozess zurückgeführt werden, wodurch sich der Wirkungsgrad des Prozesses erhöht.

Die erfindungsgemäße Ausgestaltung einer Vorrichtung zur Reinigung eines sauergashaltigen Kohlenwasserstoffstromes wird anhand einer Zeichnung genauer erläutert, wobei das erfindungsgemäße Verfahren nicht auf diese Ausführungsform beschränkt ist.

Das zu reinigende Gas (1), vorzugsweise ein Alken im Gemisch mit Wasserstoff, Alkanen, Kohlendioxid wird in eine Absorptionskolonne (2) gegeben, in der das zu reinigende Gas mit einem zugeführten chemisch wirkenden absorbierenden Lösungsmittel (3) in Form einer wässrigen Aminlösung in Kontakt gebracht wird. Dadurch erhält man einen an Kohlendioxid abgereicherten Gasstrom (4). Das an Kohlendioxid angereicherte Lösungsmittel (5) wird mittels eines Kompressors (6) am Sumpf der Kolonne abgezogen, durch einen Wärmetauscher (7) vorgewärmt und in einen als Füllkörperkolonne gearteten Hochdruck-Flash-Behälter (8) entspannt. Dort wird das beladene Lösungsmittel entspannt, so dass die absorbierten Gase in Form eines an kohlenwasserstoffreichen Gasstroms (9) entweichen. In Gegenrichtung wird ein Brenngas (10), welches Erdgas ist, durch den Hochdruck-Flash-Behälter (8) geführt. Am Sumpf des Hochdruck-Flash- Behälters (8) erhält man das kohlendioxidreiche Lösungsmittel (11), welches einer Lösungsmittelregenerationskolonne (12) zugeführt wird. Am Kopf des Hochdruck-Flash-Behälters (8) erhält man den desorbierten kohlenwasserstoffreichen Gasstrom (9) im Gemisch mit dem Erdgas, welcher in den Hauptprozessgasweg durch einen Kompressor (13) zurückgeführt wird. Am Kopf der Lösungsmittelregenerationskolonne (12) erhält man das aus dem Lösungsmittel ausgetriebene mehr oder weniger reine Kohlendioxid als desorbiertes Sauergas (14). Dieses wird vor der Weiterverwendung mit einer geeigneten Kühleinrichtung (14a) gekühlt. Am Sumpf der Lösungsmittelregenerationskolonne (12) erhält man das entspannte und desorbierte Lösungsmittel (12a) zurück. Die Lösungsmittelregenerationskolonne (12) wird zur Desorption mit einem Reboiler (12b) beheizt. Das Lösungsmittel wird im Kreis (3) geführt und vor Wiederverwendung in der Absorptionskolonne (2) über einen Wärmetauscher (7) zur Erwärmung des Ausgangslösungsmittelstromes und mit einer geeigneten Kühlvorrichtung (3a) gekühlt. Der an Kohlendioxid abgereicherte Produktstrom (4) wird zur weiteren Produktaufarbeitung einer Cold-Box *(nicht dargestellt)* zugeführt.

### Bezugszeichenliste

- 1: Zu reinigendes Gas
- 2: Absorptionskolonne
- 3: Kohlendioxid absorbierendes chemisch wirkendes Lösungsmittel (wässrige Aminlösung)
- 3a: Lösungsmittelkühler
- 4: An Kohlendioxid abgereicherter Gasstrom
- 5: An Kohlendioxid angereichertes Lösungsmittel
- 6: Kompressor
- 7: Wärmetauscher
- 8: "Hochdruck-Flash-Behälter" (Füllkörperkolonne)
- 9: Kohlenwasserstoffreicher Gasstrom
- 10: Erdgas als Brenngas
- 11: Kohlendioxidreiches Lösungsmittel
- 12: Lösungsmittelregenerationskolonne
- 12a: Desorbiertes Lösungsmittel
- 12b: Reboiler
- 13: Kompressor
- 14: Desorbiertes Sauergas
- 14a: Kondensator

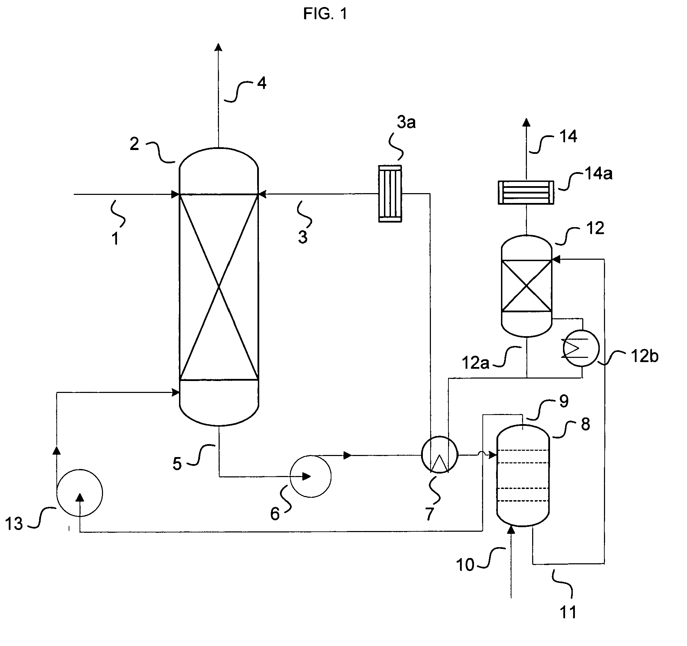

## Patentansprüche

1. Verfahren zur Entfernung von Kohlendioxid aus einem Kohlenwasserstoffstrom bei der Herstellung von Alkenen aus Alkanen, wobei
• ein Alkane und ein inertes Gas enthaltendes technisch sauerstofffreies Gasgemisch in eine mindestens ein Katalysatorbett enthaltende Reaktionsvorrichtung zur Dehydrierung des eingesetzten Alkans geleitet wird, und
• das gebildete Alkene, Alkane, Wasserstoff, das inerte Gas und Kohlendioxid (CO2) enthaltende Gasgemisch aus der Reaktionsvorrichtung abgezogen wird, und
• die im Prozessgas noch enthaltenen Sauergasbestandteile, wie Kohlendioxid, Schwefelwasserstoff (2S), Carbonylsulfid (COS) und Merkaptane in einer der Dehydrierung nachgeschalteten Gaswäsche vollständig oder nahezu vollständig mittels eines chemischen Lösungsmittels absorbiert werden,
**dadurch gekennzeichnet, dass**
• das gebildete Adsorbat, welches auch mitabsorbierte Kohlenwasserstoffe enthält, auf den Kopf eines "Hochdruck-Flash-Behälters" geleitet wird, welcher Stoffaustauschelemente enthält, und dem Sumpf des "Hochdruck-Flash-Behälters" ein Brenngas zugeführt wird, welches im Gegenstrom zu der über die Stoffaustauschelemente herabfließenden, angereicherten Absorptionslösung geführt wird, wobei der größte Teil der in der angereicherten Absorptionslösung enthaltenen Kohlenwasserstoffe und im besonderen die noch gelösten Alkene aus der Lösung desorbiert werden und den "Hochdruck-Flash-Behälter" über Kopf verlassen,
• es sich bei dem Brenngas um das Heizgas handelt, welches für die Beheizung des Dehydrierungsreaktors verwendet wird und welches abgezweigt wird, und es sich bei dem Heizgas, welches für die Beheizung des Dehydrierungsreaktors verwendet wird, um Erdgas handelt, und als Lösungsmittel für die Gaswäsche ein chemisch wirkendes Absorptionsmittel eingesetzt wird, und
• dass als chemisches Lösungsmittel für die Gaswäsche eine wässrige Aminlösung eingesetzt wird.

2. Verfahren zur Entfernung von Kohlendioxid aus einem Kohlenwasserstoffstrom bei der Herstellung von Alkenen aus Alkanen nach Anspruch 1, **dadurch gekennzeichnet, dass** den "Hochdruck-Flash-Behälter" über Kopf verlassende Gas zurück in den Prozessgasweg hinter die Dehydrierungseinrichtung zurückgeführt wird.

3. Verfahren zur Entfernung von Kohlendioxid aus einem Kohlenwasserstoffstrom bei der Herstellung von Alkenen aus Alkanen nach einem der Ansprüche 1 oder 2, **dadurch gekennzeichnet, dass** der "Hochdruck-Flash-Behälter" bei einem Druck von 1 bis 20 bar betrieben wird.

4. Verfahren zur Entfernung von Kohlendioxid aus einem Kohlenwasserstoffstrom bei der Herstellung von Alkenen aus Alkanen nach einem der Ansprüche 1 bis 3, **dadurch gekennzeichnet, dass** der "Hochdruck-Flash-Behälter" bei einer Temperatur von 40 °C bis 160 °C betrieben wird.

5. Verfahren zur Entfernung von Kohlendioxid aus einem Kohlenwasserstoffstrom bei der Herstellung von Alkenen aus Alkanen nach einem der Ansprüche 1 bis 4, **dadurch gekennzeichnet, dass** es sich bei dem Alkan um Propan und dem daraus hergestellten Alken um Propylen handelt.

6. Verfahren zur Entfernung von Kohlendioxid aus einem Kohlenwasserstoffstrom bei der Herstellung von Alkenen aus Alkanen nach einem der Ansprüche 1 bis 5, **dadurch gekennzeichnet, dass** es sich bei dem inerten Gas in der Dehydrierungsreaktion um Wasserdampf handelt.

7. Verfahren zur Entfernung von Kohlendioxid aus einem Kohlenwasserstoffstrom bei der Herstellung von Alkenen aus Alkanen nach einem der Ansprüche 1 bis 6, **dadurch gekennzeichnet, dass** als Lösungsmittel für die Gaswäsche eine wässrige Monoethanolaminlösung eingesetzt wird.

8. Verfahren zur Entfernung von Kohlendioxid aus einem Kohlenwasserstoffstrom bei der Herstellung von Alkenen aus Alkanen nach einem der Ansprüche 1 bis 6, **dadurch gekennzeichnet, dass** als Lösungsmittel für die Gaswäsche eine wässrige Methyldiethanolaminlösung eingesetzt wird.

9. Verfahren zur Entfernung von Kohlendioxid aus einem Kohlenwasserstoffstrom bei der Herstellung von Alkenen aus Alkanen nach einem der Ansprüche 7 oder 8, **dadurch gekennzeichnet, dass** das Lösungsmittel für die Gaswäsche einen Aktivator aus der Gruppe der primären und sekundären Amine enthält.

10. Verfahren zur Entfernung von Kohlendioxid aus einem Kohlenwasserstoffstrom bei der Herstellung von Alkenen aus Alkanen nach einem der Ansprüche 1 bis 9, **dadurch gekennzeichnet, dass** das von Sauergasen befreite Produktgas einer "Cold-Box" und einer Rückgewinnungskolonne zur weiteren Rückgewinnung von noch im Prozessgas enthalten Alkenen zugeführt wird.

11. Vorrichtung zur Entfernung von Kohlendioxid aus einem Kohlenwasserstoffstrom bei der Herstellung von Alkenen aus Alkanen, wobei diese eine Absorptionskolonne enthält, die eine Gaswäsche durch Inkontaktnahme mit einem sauergasabsorbierenden Lösungsmittel ermöglicht, **dadurch gekennzeichnet, dass** der Absorptionskolonne ein "Hochdruck-Flash-Behälter" nachgeschaltet ist, der eine Entspannung des sauergasbeladenen Lösungsmittels ermöglicht, und dieser Behälter einen Aufgabestutzen für das sauergasbeladene Lösungsmittel, einen Aufgabestutzen für ein Brenngas, einen Entnahmestutzen für ein sauergasbeladenes Lösungsmittel und einen Entnahmestutzen für einen gasförmigen Kohlenwasserstoffstrom enthält.

## Claims

1. A process for removing carbon dioxide from a hydrocarbon stream during the manufacture of alkenes from alkanes, wherein
• a technically oxygen-free gas mixture containing alkanes and an inert gas is directed into a reaction facility containing at least one catalyst bed for dehydrating the used alkane, and
• the gas mixture containing formed alkenes, alkanes, hydrogen, the inert gas and carbon dioxide (CO2) is removed from the reaction facility, and
• the acid gas constituents remaining in the process gas, such as carbon dioxide, hydrogen sulphide (2S), carbonyl sulphide (COS) and mercaptanes are completely or almost completely absorbed in a gas scrubbing arranged downstream from the dehydration by means of a chemical solvent,
**characterised in that**
• the formed adsorbate also containing co-absorbed hydrocarbons is directed to the head of a "high-pressure flash vessel", which contains material exchange elements, and a combustion gas is fed into the sump of the "high-pressure flash vessel", which is conducted in a counter-current to the enriched absorption solution flowing down across the material exchange elements, wherein the largest part of the hydrocarbons contained in the enriched absorption solution and, in particular, the still dissolved alkenes, are desorbed from the solution and leave via the head of the "high-pressure flash vessel",
• the combustion gas is a heating gas, which is used for heating the dehydrating reactor and which is branched off, and wherein the heating gas, which is used for heating the dehydrating reactor, is natural gas, and the solvent used in the gas scrubbing is a chemically effective absorption agent, and
• **in that** the chemical solvent used for the gas scrubbing is an aqueous amine solution.

2. The process for removing carbon dioxide from a hydrocarbon stream during the manufacture of alkenes from alkanes according to claim 1, **characterised in that** the gas leaving via the head of the "high-pressure flash vessel" is fed back into the process gas path behind the dehydrating facility.

3. The process for removing carbon dioxide from a hydrocarbon stream during the manufacture of alkenes from alkanes according to one of claims 1 or 2, **characterised in that** the "high-pressure flash vessel" is operated at a pressure from 1 to 20 bar.

4. The process for removing carbon dioxide from a hydrocarbon stream during the manufacture of alkenes from alkanes according to one of claims 1 to 3, **characterised in that** the "high-pressure flash vessel" is operated at a temperature from 40°C to 160°C.

5. The process for removing carbon dioxide from a hydrocarbon stream during the manufacture of alkenes from alkanes according to one of claims 1 to 4, **characterised in that** the alkane is propane and the alkene produced from it is propylene.

6. The process for removing carbon dioxide from a hydrocarbon stream during the manufacture of alkenes from alkanes according to one of claims 1 to 5, **characterised in that** the inert gas in the dehydrating reaction is water vapour.

7. The process for removing carbon dioxide from a hydrocarbon stream during the manufacture of alkenes from alkanes according to one of claims 1 to 6, **characterised in that** the solvent used in the gas scrubbing is an aqueous monoethanolamine solution.

8. The process for removing carbon dioxide from a hydrocarbon stream during the manufacture of alkenes from alkanes according to one of claims 1 to 6, **characterised in that** the solvent used in the gas scrubbing is an aqueous methyldiethanolamine solution.

9. The process for removing carbon dioxide from a hydrocarbon stream during the manufacture of alkenes from alkanes according to one of claims 7 or 8, **characterised in that** the solvent used in the gas scrubbing is an activator from the group of primary and secondary amines.

10. The process for removing carbon dioxide from a hydrocarbon stream during the manufacture of alkenes from alkanes according to one of claims 1 to 9, **characterised in that** the product gas freed from acid gases is directed to a "cold box" and to a recovery column for recovering any remaining alkenes still present in the process gas.

11. An apparatus for removing carbon dioxide from a hydrocarbon stream during the manufacture of alkenes from alkanes, wherein this includes an absorption column, which permits gas scrubbing by making contact with an acid gas absorbing solvent, **characterised in that** the absorption column has a "high-pressure flash vessel" arranged downstream from it, which allows the acid-gas-laden solvent to be relaxed, and this vessel contains a charge connection piece for the acid-gas-laden solvent, a charge connection piece for a combustion gas, a discharge connection piece for an acid-gas-laden solvent and a discharge connection piece for a gaseous hydrocarbon stream.

## Revendications

1. Procédé pour éliminer du dioxyde de carbone d'un flux d'hydrocarbures lors de la production d'alcènes à partir d'alcanes, dans lequel
• un mélange de gaz techniquement exempt d'oxygène contenant des alcanes et un gaz inerte est conduit dans un dispositif de réaction contenant au moins un lit de catalyseur pour la déshydrogénation de l'alcane utilisé, et
• le mélange de gaz formé, contenant des alcènes, des alcanes, de l'hydrogène, le gaz inerte et du dioxyde de carbone (CO2), étant soustrait du dispositif de réaction, et
• les composants gazeux acides se trouvant encore dans le gaz de traitement, tels le dioxyde de carbone, l'hydrogène sulfuré (2S), le sulfure de carbonyle (COS) et les mercaptans étant entièrement, ou presque entièrement absorbés au moyen d'un solvant chimique dans un lavage de gaz monté en aval de la déshydrogénation,
**caractérisé en ce que**
• l'adsorbat formé, lequel contient également des hydrocarbures absorbés avec, est conduit sur la tête d'un « récipient flash à haute pression » qui contient des éléments de transfert de matière, et un gaz combustible étant amené au puisard du « récipient flash à haute pression », lequel gaz est conduit à contre-courant de la solution d'absorption enrichie circulant vers le bas via les éléments de transfert de matière, dans lequel la plus grande partie des hydrocarbures contenus dans la solution d'absorption enrichie, et en particulier les alcènes encore dissous, sont désorbés et quittent le « récipient flash à haute pression » par la tête,
• le gaz combustible étant du gaz de chauffage qui est utilisé pour le chauffage du réacteur de déshydrogénation et qui est séparé, et le gaz de chauffage qui est utilisé pour le chauffage du réacteur de déshydrogénation étant du gaz naturel, et un moyen d'absorption à effet chimique étant utilisé en tant que solvant pour le lavage de gaz, et
• une solution aqueuse d'amine étant utilisée en tant que solvant chimique pour le lavage de gaz.

2. Procédé pour éliminer du dioxyde de carbone d'un flux d'hydrocarbures lors de la production d'alcènes à partir d'alcanes selon la revendication 1, **caractérisé en ce que** le gaz quittant le « récipient flash à haute pression » par la tête est ramené dans le trajet de gaz de traitement derrière le dispositif de déshydrogénation.

3. Procédé pour éliminer du dioxyde de carbone d'un flux d'hydrocarbures lors de la production d'alcènes à partir d'alcanes selon la revendication 1 ou 2, **caractérisé en ce que** le « récipient flash à haute pression » est exploité avec une pression de 1 à 20 bars.

4. Procédé pour éliminer du dioxyde de carbone d'un flux d'hydrocarbures lors de la production d'alcènes à partir d'alcanes selon l'une des revendications 1 à 3, **caractérisé en ce que** le « récipient flash à haute pression » est exploité avec une température de 40°C à 160°C.

5. Procédé pour éliminer du dioxyde de carbone d'un flux d'hydrocarbures lors de la production d'alcènes à partir d'alcanes selon l'une des revendications 1 à 4, **caractérisé en ce que**, concernant l'alcane, il s'agit de propane et concernant l'alcène produit à partir de celui-ci, il s'agit de propylène.

6. Procédé pour éliminer du dioxyde de carbone d'un flux d'hydrocarbures lors de la production d'alcènes à partir d'alcanes selon l'une des revendications 1 à 5, **caractérisé en ce que**, concernant le gaz inerte dans la réaction de déshydrogénation, il s'agit de vapeur d'eau.

7. Procédé pour éliminer du dioxyde de carbone d'un flux d'hydrocarbures lors de la production d'alcènes à partir d'alcanes selon l'une des revendications 1 à 6, **caractérisé en ce que** l'on utilise une solution aqueuse de monoéthanolamine en guise de solvant pour le lavage de gaz.

8. Procédé pour éliminer du dioxyde de carbone d'un flux d'hydrocarbures lors de la production d'alcènes à partir d'alcanes selon l'une des revendications 1 à 6, **caractérisé en ce que** l'on utilise une solution aqueuse de méthyldiéthanolamine en guise de solvant pour le lavage de gaz.

9. Procédé pour éliminer du dioxyde de carbone d'un flux d'hydrocarbures lors de la production d'alcènes à partir d'alcanes selon l'une des revendications 7 ou 8, **caractérisé en ce que** le solvant pour le lavage de gaz contient un activateur du groupe des amines principaux et secondaires.

10. Procédé pour éliminer du dioxyde de carbone d'un flux d'hydrocarbures lors de la production d'alcènes à partir d'alcanes selon l'une des revendications 1 à 9, **caractérisé en ce que** le gaz produit débarrassé des gaz acides est amené vers une « cold-box » et une colonne de récupération pour la poursuite de la récupération d'alcènes encore contenus dans le gaz de traitement.

11. Dispositif pour éliminer du dioxyde de carbone d'un flux d'hydrocarbures lors de la production d'alcènes à partir d'alcanes, dans lequel celui-ci contient une colonne d'absorption qui permet un lavage de gaz par l'entrée en contact avec un solvant absorbant les gaz acides, **caractérisé en ce qu'**un « récipient flash à haute pression » est monté en aval de la colonne d'absorption, lequel permet une détente du solvant chargé en gaz acides, et **en ce que** ce récipient contient un raccord d'alimentation pour le solvant chargé en gaz acide, un raccord d'alimentation pour un gaz combustible, un raccord de soutirage pour un solvant chargé en gaz acide et un raccord de soutirage pour un flux d'hydrocarbures gazeux.
